# EUROPEAN PATENT APPLICATION

(11) **EP 4 834 237 A1**
(43) Date of publication of application: **04.11.2026**
(21) Application number: 25173834.0
(22) Date of filing: 01.05.2025
(51) Int. Cl.: A61B 8/08, A61B 8/00

(54) **METHODS AND SYSTEMS FOR OUT-OF-PLANE VESSEL ACCESS GUIDANCE**

(71) Applicant: Koninklijke Philips N.V., 5656 AE Eindhoven (NL)
(72) Inventor: ERKAMP, Ramon Quido, Eindhoven (NL); MUKADDIM, Rashid Al, Eindhoven (NL); CHEN, Alvin, 5656AG Eindhoven (NL); RAJU, Balasundar Iyyavu, Eindhoven (NL)
(74) Representative: Philips Intellectual Property & Standards

(57) **Abstract**

A method (100) comprising: receiving (112) ultrasound imaging of a target blood vessel; receiving (114) information about an initial elevational angle and lateral angle of an ultrasound imaging probe; receiving (116) an identification of a location of the target blood vessel in the received ultrasound imaging; adjusting (118) the elevational angle of the probe toward a needle; receiving (120) an updated elevational angle and lateral angle of the probe; receiving (122) a lateral coordinate and depth coordinate for the detected tip of the needle; generating (124) a trajectory between the second position and the identified location of the target blood vessel; adjusting (126) the elevational angle of the ultrasound probe away from the tip of the needle and toward the target blood vessel; displaying (128) the generated trajectory; receiving (134) final ultrasound imaging; and determining (136) that the tip of the needle is in the target blood vessel.

## Description

### FIELD OF THE INVENTION

The present disclosure is directed generally to methods and systems for guidance of needle access to a target blood vessel using a vascular access system.

### BACKGROUND OF THE INVENTION

Ultrasound technology has become a ubiquitous and indispensable tool in modern medicine. Its widespread adoption is due to its non-invasive nature, real-time imaging capabilities, and versatility in diagnosing and monitoring a variety of medical conditions. Ultrasound exams are routinely performed to assess fetal development, guide interventions, and provide critical information that aids in ensuring the health and well-being of both mother and child. Beyond obstetrics, ultrasound is used in cardiology, emergency medicine, and many other specialties, making it an essential component of contemporary healthcare practices. Its ability to deliver immediate, clear images without the risks associated with ionizing radiation further underscores its utility and widespread use in clinical settings worldwide.

Ultrasound guided needle insertion is a standard of care practice with many applications including vascular access. In vascular access procedures, one of the critical components is to perform accurate needle insertion to the blood vessel for cannulation. When the target vessel is an artery, inaccurate cannulation can result in leaking of blood through wall of the vessel, possibly creating a pseudoaneurysm or aneurysm by disrupting the vessel walls.

One example of a vascular access application is a life-saving procedure at a point of injury called Resuscitative Endovascular Balloon Occlusion of the Aorta (REBOA), which addresses preventable hemorrhage-related mortality. However, the most challenging aspect of applying endovascular resuscitative techniques for hemorrhage control is early common femoral artery (CFA) access. Without a method to guarantee femoral access in austere and resource-limited locations, the translation of endovascular interventions to prehospital and early point of care settings cannot be fully realized.

Currently, vascular access protocols are often limited to expert applications, thus creating a significant barrier for the widespread adoption of these lifesaving procedures. Therefore, it is critical to develop a solution which can enable reliable vascular access for both novice and expert users.

### SUMMARY OF THE INVENTION

There is thus a continued need for improved methods and systems to guide needle access to a target blood vessel, for both novices and experts alike.

Various embodiments and implementations are directed to methods and systems for guiding needle access to a target blood vessel using a vascular access system. The system receives ultrasound imaging including ultrasound imaging of the target blood vessel, while a tip of the needle is positioned at a first position relative to the target blood vessel, and receives information about an initial elevational angle and an initial lateral angle of the ultrasound imaging probe. After the needle is moved slightly to a second position toward the location of the target blood vessel, the elevational angle of the ultrasound probe is adjusted toward the needle. The system receives an updated elevational angle and an updated lateral angle of the ultrasound imaging probe, and generates - using the updated elevational angle, the updated lateral angle, the lateral coordinate of the detected tip of the needle, the depth coordinate of the detected tip of the needle, and the identified location of the target blood vessel - a trajectory between the second position and the identified location of the target blood vessel. The elevational angle of the ultrasound probe is again adjusted, this time away from the tip of the needle and toward the target blood vessel. The generated trajectory is displayed on a user interface. After the tip of the needle is moved to a third position based on the displayed generated trajectory, the system receives final ultrasound imaging and determines from that final ultrasound imaging that the tip of the needle is in the target blood vessel.

The invention is defined by the independent claims. Advantageous embodiments are provided in the dependent claims.

According to an aspect, a method for guiding needle access to a target blood vessel is provided. The method includes: (i) receiving, from an ultrasound imaging probe of a vascular access system, ultrasound imaging including ultrasound imaging of the target blood vessel, while a tip of the needle is positioned at a first position relative to the target blood vessel; (ii) receiving, from one or more sensors of the vascular access system, information about an initial elevational angle and an initial lateral angle of the ultrasound imaging probe at the time of obtaining the received ultrasound imaging; (iii) receiving an identification of a location of the target blood vessel in the received ultrasound imaging; (iv) adjusting, after the tip of the needle is moved to a second position toward the location of the target blood vessel, the elevational angle of the ultrasound probe toward the needle while obtaining updated ultrasound imaging, until the tip of the needle is detected in the updated ultrasound imaging; (v) receiving an updated elevational angle and an updated lateral angle of the ultrasound imaging probe at the time of obtaining the updated ultrasound imaging; (vi) receiving a lateral coordinate and depth coordinate for the detected tip of the needle; (vii) generating, using the updated elevational angle, the updated lateral angle, the lateral coordinate of the detected tip of the needle, the depth coordinate of the detected tip of the needle, and the identified location of the target blood vessel, a trajectory between the second position and the identified location of the target blood vessel; (viii) adjusting, after the trajectory between the second position and the identified location of the target blood vessel is generated, the elevational angle of the ultrasound probe away from the tip of the needle and toward the target blood vessel; (ix) displaying the generated trajectory on a user interface, the generated trajectory comprising a needle location marker; (x) receiving, after the tip of the needle is moved to a third position based on the displayed generated trajectory, final ultrasound imaging; and (xi) determining, from the final ultrasound imaging, that the tip of the needle is in the target blood vessel.

According to an embodiment, receiving an identification of a location of the target blood vessel in the received ultrasound imaging comprises receiving an identification from a clinician via a user interface.

According to an embodiment, receiving an identification of a location of the target blood vessel in the received ultrasound imaging comprises an identification of the target blood vessel by an ultrasound algorithm.

According to an embodiment, receiving ultrasound imaging including ultrasound imaging of the target blood vessel comprises receiving a sweep of a region comprising the target blood vessel.

According to an embodiment, an aperture of the ultrasound probe is kept at a same location while the elevational angle of the ultrasound probe is adjusted toward the needle.

According to an embodiment, the method further includes reporting to the clinician via the user interface, the determination that the tip of the needle is in the target blood vessel.

According to an embodiment, the method further includes: determining, after the elevational angle of the ultrasound probe is adjusted away from the tip of the needle and toward the target blood vessel, that the adjustment exceeds a predetermined threshold of the elevational angle; and providing, via the user interface, an indication that the adjustment exceeds a predetermined threshold of the elevational angle.

According to an embodiment, the steps of adjusting, displaying, and receiving are repeated until the step of determining that the tip of the needle is in the target blood vessel.

According to another aspect, a vascular access system configured to enable needle access to a target blood vessel is provided. The system includes a needle comprising a needle tip; an ultrasound probe configured to obtain ultrasound imaging including ultrasound imaging of the target blood vessel, while the tip of the needle is positioned at a first position relative to the target blood vessel; one or more sensors configured to obtain information about an initial elevational angle and an initial lateral angle of the ultrasound imaging probe at the time of obtaining ultrasound imaging; a user interface; and a processor configured to: (i) receive, via the ultrasound probe, ultrasound imaging including ultrasound imaging of the target blood vessel; (ii) receive, from the one or more sensors, information about an initial elevational angle and an initial lateral angle of the ultrasound imaging probe at the time of obtaining ultrasound imaging; (iii) receive an identification of a location of the target blood vessel in the received ultrasound imaging; (iv) adjust, after the tip of the needle is moved to a second position toward the location of the target blood vessel, the elevational angle of the ultrasound probe toward the needle while obtaining updated ultrasound imaging, until the tip of the needle is detected in the updated ultrasound imaging; (v) receive an updated elevational angle and an updated lateral angle of the ultrasound imaging probe at the time of obtaining the updated ultrasound imaging; (vi) receive a lateral coordinate and depth coordinate for the detected tip of the needle; (vii) generate, using the updated elevational angle, the updated lateral angle, the lateral coordinate of the detected tip of the needle, the depth coordinate of the detected tip of the needle, and the identified location of the target blood vessel, a trajectory between the second position and the identified location of the target blood vessel; (viii) adjust, after the trajectory between the second position and the identified location of the target blood vessel is generated, the elevational angle of the ultrasound probe away from the tip of the needle and toward the target blood vessel; (ix) display the generated trajectory on the user interface; (x) receive, after the tip of the needle is moved to a third position based on the displayed generated trajectory, final ultrasound imaging; and (xi) determine, from the final ultrasound imaging, that the tip of the needle is in the target blood vessel.

According to an embodiment, receiving an identification of a location of the target blood vessel in the received ultrasound imaging comprises receiving an identification from a clinician via a user interface.

According to an embodiment, receiving an identification of a location of the target blood vessel in the received ultrasound imaging comprises an identification of the target blood vessel by an ultrasound algorithm.

According to an embodiment, receiving ultrasound imaging including ultrasound imaging of the target blood vessel comprises receiving a sweep of a region comprising the target blood vessel.

According to an embodiment, an aperture of the ultrasound probe is kept at a same location while the elevational angle of the ultrasound probe is adjusted toward the needle.

According to an embodiment, the user interface is further configured to report to the clinician the determination that the tip of the needle is in the target blood vessel.

According to an embodiment, the processor is further configured to: determine, after the elevational angle of the ultrasound probe is adjusted away from the tip of the needle and toward the target blood vessel, that the adjustment exceeds a predetermined threshold of the elevational angle; and provide, via the user interface, an indication that the adjustment exceeds a predetermined threshold of the elevational angle.

According to an embodiment, the processor is further configured to repeat the steps of adjusting, displaying, and receiving until the step of determining that the tip of the needle is in the target blood vessel.

According to yet another aspect, a non-transitory machine-readable medium is provided. The medium stores instructions which, when executed by a processor of a vascular access system according to any of the above embodiments, cause the processor to perform the method for needle access to a target blood vessel according to any of the embodiments described above.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
Fig. 1A is a flowchart of a method for needle access to a target blood vessel, in accordance with an embodiment.
Fig. 1B is a flowchart of a method for needle access to a target blood vessel, in accordance with an embodiment.
Fig. 2 is a schematic representation of a vascular access system, in accordance with an embodiment.
Fig. 3 is a is a schematic representation of out-of-plane vessel access using elevational probe tilting to track progression of the needle, in accordance with an embodiment.
Fig. 4 is a flowchart of a method for guiding needle access to a target blood vessel using a vascular access system, in accordance with an embodiment.
Fig. 5 is a schematic representation of a user interface showing 3D probe needle guidance, in accordance with an embodiment.

### DETAILED DESCRIPTION OF EMBODIMENTS

The present disclosure describes various embodiments of a system and method configured to guide needle access to a target blood vessel using a vascular access system. More generally, Applicant has recognized and appreciated that it would be beneficial to more efficiently and accurately enable both novices and experts to guide a needle tip to a target blood vessel. Thus, a vascular access system receives ultrasound imaging including ultrasound imaging of the target blood vessel, while a tip of the needle is positioned at a first position relative to the target blood vessel, and receives information about an initial elevational angle and an initial lateral angle of the ultrasound imaging probe. After the needle is moved slightly to a second position toward the location of the target blood vessel, the elevational angle of the ultrasound probe is adjusted toward the needle. The system receives an updated elevational angle and an updated lateral angle of the ultrasound imaging probe, and generates - using the updated elevational angle, the updated lateral angle, the lateral coordinate of the detected tip of the needle, the depth coordinate of the detected tip of the needle, and the identified location of the target blood vessel - a trajectory between the second position and the identified location of the target blood vessel. The elevational angle of the ultrasound probe is again adjusted, this time away from the tip of the needle and toward the target blood vessel. The generated trajectory is displayed on a user interface. After the tip of the needle is moved to a third position based on the displayed generated trajectory, the system receives final ultrasound imaging and determines from that final ultrasound imaging that the tip of the needle is in the target blood vessel.

The embodiments and implementations disclosed or otherwise envisioned herein can be utilized with any system or process that may utilize or benefit from improved guidance of a needle to a target blood vessel. The embodiments and implementations disclosed or otherwise envisioned herein can be utilized with any system that is used to guide a needle, including but not limited to Philips^{®} Lumify^{®} (manufactured by Koninklijke Philips, N.V.), among other products. However, the disclosure is not limited to these devices or systems, and thus the disclosure and embodiments disclosed herein can encompass any system that may utilize or benefit from improved guidance of a needle to a target blood vessel.

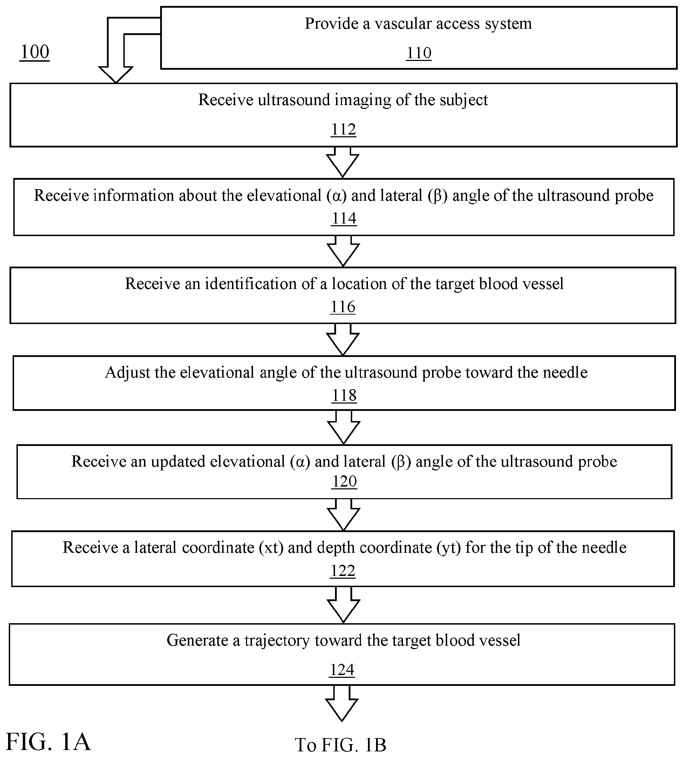

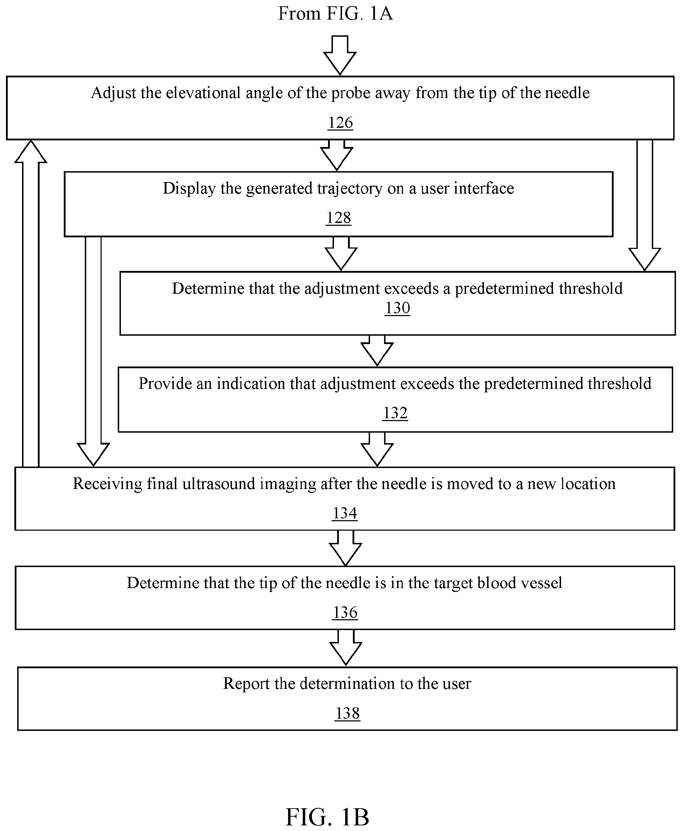

Referring to Fig. 1 (split into Figs. 1A and 1B), in one embodiment, is a flowchart of a method 100 for guiding needle access to a target blood vessel using a vascular access system. The methods described in connection with the figures are provided as examples only, and shall be understood not to limit the scope of the disclosure. The vascular access system can be any of the systems described or otherwise envisioned herein. The vascular access system can be a single system or multiple different systems.

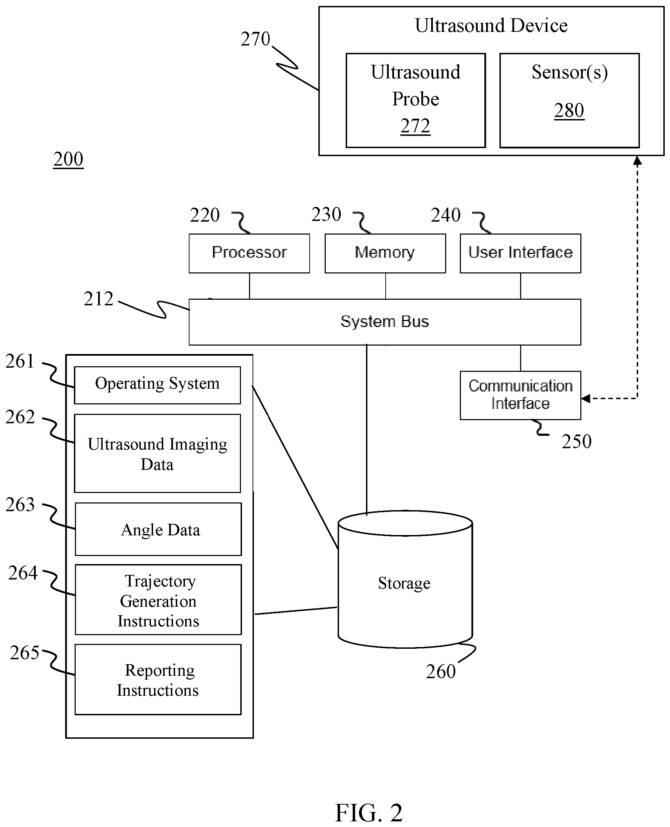

At step 110 of the method, a vascular access system 200 is provided. Referring to an embodiment of a vascular access system 200 as depicted in Fig. 2, for example, the system comprises one or more of a processor 220, memory 230, user interface 240, communications interface 250, and storage 260, interconnected via one or more system buses 212. It will be understood that Fig. 2 constitutes, in some respects, an abstraction and that the actual organization of the components of the system 200 may be different and more complex than illustrated. Additionally, vascular access system 200 can be any of the systems described or otherwise envisioned herein. Other elements and components of the vascular access system 200 are disclosed and/or envisioned elsewhere herein.

According to an embodiment, the vascular access system 200 comprises or is in direct or indirect communication with an imaging modality 270. The imaging modality can be, for example, an ultrasound system such as EPIQ^{®} system, Philips Lumify^{®}, and Philips Affinity^{®}, among many others. The images obtained using the imaging modality may be obtained from a clinical provider or other individual. For example, the user of the imaging modality may be an experienced sonographer, a first-time user of ultrasound, or any experience level in between. As another example, the imaging modality is a handheld ultrasound probe connected to a mobile computing device such as a smartphone or tablet, among other mobile computing devices.

At step 112 of the method, ultrasound imaging of the patient or subject is obtained using the imaging modality 270 of the vascular access system 200. The ultrasound imaging input is obtained using an ultrasound imaging probe 272 of the vascular access system 200. According to an embodiment, the ultrasound imaging is 2D B-mode imaging, capable of obtaining a cross-sectional image - and/or other image - of the target blood vessel. The ultrasound imaging includes ultrasound imaging of the target blood vessel, including while a tip of a needle is positioned at a first position relative to the target blood vessel. The first position can be any position, inside or outside the body of the patient. The needle is any needle or device that will be inserted into, e.g., puncturing, a target such as a target blood vessel. The target can be any target in the patient's body, including a target blood vessel in the patient. The patient or subject can be any subject that could potentially benefit from ultrasound. Thus, the ultrasound imaging may be obtained of any region of the subject's body, such as the abdomen, chest, head, or limbs among other regions. The ultrasound imaging may be obtained using any method, system, or technique for obtaining ultrasound imaging.

Once obtained, the ultrasound imaging may be utilized immediately, and/or it may be temporarily or permanently stored in local and/or remote memory for future use, including in an ultrasound imaging database.

At step 114 of the method, the vascular access system 200 receives information about the elevational (α) angle and lateral (β) angle of the imaging plane, i.e., of the ultrasound imaging probe 272 at the time of obtaining the received ultrasound imaging. The vascular access system receives the angle information from one or more sensors 280. The one or more sensors can be any sensors capable of generating and providing information about the elevational (α) angle and lateral (β) angle of the imaging plane.

According to one embodiment, the information about the elevational (α) angle and lateral (β) angle can be based on inclinometers that measure tip and tilt based on gravity, optical methods, mechanical methods, or by electronically steering a 2D imaging plane of a 3D ultrasound probe, among other methods. For example, the system such as the ultrasound probe can comprise an inertial measurement unit (IMU), using one or more of an accelerometer, gyroscope, and magnetometer.

According to an embodiment, the information about the elevational (α) angle and lateral (β) angle is obtained at least in part using a gravity sensor. Gravity sensors are commonplace in low cost digital level tools for construction and in cellphones, and have measurement accuracy better than 0.2 degrees. According to an embodiment, the information about the elevational (α) angle and lateral (β) angle is obtained at least in part using a contact plate. For example, a contact plate can be added around part of the perimeter of the probe aperture, and attached to the probe with a pivoting mechanical connection that has angle sensors. According to an embodiment, the information about the elevational (α) angle and lateral (β) angle is obtained at least in part using an optical camera. A camera can be attached to the upper part of the ultrasound probe, looking down onto the arm. The camera can be a depth-imaging capable camera to determine the orientation of the arm relative to the camera. The system can use one or more conventional optical cameras to detect how the location of static landmarks in the environment (ceiling tiles, door frames, deliberately placed optical markers, etc.) are changing position, in order to accurately determine the rotational orientation of the ultrasound probe relative to the environment.

According to an embodiment, when the ultrasound imaging plane is positioned at the desired vessel puncture cross section, the current elevational and lateral imaging plane position are set as the reference orientation, i.e. α=0, β=0.

Once obtained, the elevational (α) angle and lateral (β) angle may be utilized immediately, and/or they may be temporarily or permanently stored in local and/or remote memory for future use.

At step 116 of the method, the vascular access system 200 receives an identification of a location of the target, such as a target blood vessel, in the received ultrasound imaging. The target can be the location of the targeted puncture by the tip of the needle. According to an embodiment, when the optimal vessel puncture location is visualized, a clinician interacts with the system to identify the location of the target. The clinician can interact with a user interface of the vascular access system to identify the location of the target. For example, the clinician can push a button, or select a target with a mouse, finger, or other interaction mechanism. Many methods for selecting a target in ultrasound imaging are possible.

According to another embodiment, the target blood vessel and/or the optimal vessel puncture location is identified using a targeting or identification algorithm. Many ultrasound targeting or identification algorithms are known, and can be used for identifying the target blood vessel and/or the optimal vessel puncture location.

Once the target blood vessel and/or the optimal vessel puncture location are identified manually and/or using an algorithm, the system has or obtains the lateral coordinates of the target (xt) and the depth coordinates of the target (yt). According to an embodiment, the lateral coordinates of the vessel (xt) and the depth coordinates of the target vessel (yt) in the imaging plane are stored (xt, yt). Thus, once obtained, the lateral coordinates (xt) and depth coordinates (yt) may be utilized immediately, and/or they may be temporarily or permanently stored in local and/or remote memory for future use.

Next, the needle is advanced, preferably toward the target. This can include puncturing the patient, or advancing the needle toward the target if the needle has already punctured the patient. The needle advancement can be relatively minor, on the order of millimeters or centimeters. The needle advancement can be a manual advancement by a clinician, or can be an automated advancement by the vascular access system 200.

Thus, according to an embodiment, the tip of the needle is moved from an initial first position to a new, second position, preferably toward the location of the target blood vessel. At step 118 of the method, after the needle is moved from an initial first position to a second position, the elevational angle of the ultrasound probe is adjusted toward the needle while obtaining updated ultrasound imaging. The aperture of the ultrasound probe is kept at the same location while the elevational angle of the ultrasound probe is adjusted toward the needle. According to an embodiment, the elevational angle of the ultrasound probe is adjusted until the tip of the needle is detected in the updated ultrasound imaging. According to an embodiment, the adjustment can be a manual adjustment by a clinician, or can be an automated adjustment by the vascular access system 200.

According to an embodiment, adjusting the elevational angle of the ultrasound probe while keeping the aperture of the ultrasound probe at the same location is different from a conventional workflow where the probe would be translated at constant tilt towards the needle. Tilting the probe instead of translating it offers several advantages. For example, when tilting the probe instead of translating it, the angle between the scanning plane and the needle shaft gets closer to orthogonal and therefore the needle visibility is significantly improved.

According to an embodiment, as soon as the tip of the needle is detected in the updated ultrasound imaging while or after tilting the elevational angle of the probe, the method proceeds to the next step.

At step 120 of the method, the vascular access system 200 receives an updated elevational angle (α) and an updated lateral angle (β) of the ultrasound probe at the new position. As described above, the updated angles can be based on or otherwise received from inclinometers that measure tip and tilt based on gravity, optical methods, mechanical methods, or by electronically steering a 2D imaging plane of a 3D ultrasound probe, among other methods. Once obtained, the updated elevational angle (α) and updated lateral angle (β) may be utilized immediately, and/or may be temporarily or permanently stored in local and/or remote memory for future use.

At step 122 of the method, which can be simultaneous with step 120 or just before or after step 120 of the method, the vascular access system 200 receives an updated lateral coordinate (xt) and updated depth coordinate (yt) for the detected tip of the needle, obtained from the updated ultrasound imaging, after adjusting the elevational angle of the ultrasound probe while keeping the aperture of the ultrasound probe at the same location. Once obtained, the updated lateral coordinates (xt) and depth coordinates (yt) may be utilized immediately, and/or may be temporarily or permanently stored in local and/or remote memory for future use.

At step 124 of the method, the vascular access system 200 generates a trajectory between the second position of the needle tip and the identified location of the target blood vessel. According to an embodiment, the system generates the trajectory using the updated elevational angle (α), updated lateral angle (β), updated lateral coordinate (xt) of the detected tip of the needle, the updated depth coordinate (yt) of the detected tip of the needle, and the identified location of the target blood vessel. According to an embodiment, the generated trajectory comprises a needle location marker. Once generated, the trajectory may be utilized immediately, and/or may be temporarily or permanently stored in local and/or remote memory for future use.

At step 126 of the method, after the trajectory between the second position and the identified location of the target blood vessel is generated, the elevational angle (α) of the ultrasound probe is adjusted away from the tip of the needle and toward the target blood vessel. The aperture of the ultrasound probe is kept at the same location while the elevational angle of the ultrasound probe is adjusted away from the needle. According to an embodiment, the adjustment can be a manual adjustment by a clinician, or can be an automated adjustment by the vascular access system 200.

At step 128 of the method, the generated trajectory is displayed on a user interface. The displayed generated trajectory can include, for example, the needle location marker among other information. The system may provide the generated trajectory to a user - such as a patient or a clinician - via any mechanism, including but not limited to a visual display or any other method of notification. The information may be communicated by wired and/or wireless communication to another device. For example, the system may communicate the information to a mobile phone, computer, laptop, wearable device, and/or any other device configured to allow display and/or other communication of the information. According to one embodiment, the user interface is a component of the vascular access system 200. According to another embodiment, the user interface is a component of a device or system separate from the vascular access system 200.

At optional step 130 of the method, after the elevational angle of the ultrasound probe is adjusted away from the tip of the needle and toward the target blood vessel, the vascular access system 200 determines that the adjustment exceeds a predetermined threshold of the elevational angle. For example, the system can compare the updated elevational angle of the ultrasound probe to the predetermined threshold and determine that the updated elevational angle exceeds, meets, or does not meet the predetermined threshold. The predetermined threshold can be determined in a wide variety of ways, including but not limited to manually programming or selecting or otherwise inputting the predetermined threshold. According to another embodiment, the predetermined threshold is determined experimentally, or is determined or set automatically by the system. Other methods for identifying, providing, selecting, or determining the predetermined threshold for the elevation angle of the ultrasound probe are possible.

At optional step 132 of the method, after determining that the adjustment exceeds a predetermined threshold of the elevational angle, the system can provide the determination to a user - such as a patient or a clinician - via any mechanism, including but not limited to a visual display or any other method of notification. The information may be communicated by wired and/or wireless communication to another device. For example, the system may communicate the information to a mobile phone, computer, laptop, wearable device, and/or any other device configured to allow display and/or other communication of the information. According to one embodiment, the user interface is a component of the vascular access system 200. According to another embodiment, the user interface is a component of a device or system separate from the vascular access system 200.

Next, the needle is again advanced toward the target. The needle advancement can be relatively minor, on the order of millimeters or centimeters, or can be a larger advancement. The needle advancement can be a manual advancement by a clinician, or can be an automated advancement by the vascular access system 200. Thus, according to an embodiment, the tip of the needle is moved from the second position to a new, third position, preferably toward the location of the target blood vessel. According to an embodiment, the tip of the needle is moved to the third position based on the displayed generated trajectory.

At step 134 of the method, after the tip of the needle is moved to the third position based on the displayed generated trajectory, the vascular access system 200 receives or obtains final ultrasound imaging. According to an embodiment, the ultrasound imaging of the patient or subject is obtained using the ultrasound imaging probe 272 of the vascular access system 200. Once obtained, the ultrasound imaging may be utilized immediately, and/or may be temporarily or permanently stored in local and/or remote memory for future use, including in an ultrasound imaging database.

Notably, according to an embodiment, the steps of adjusting (126), displaying (128), and receiving (134) are repeated until the system determines that the tip of the needle is in the target blood vessel. Thus, the system returns to step 126 (adjusting the elevational angle (α) of the ultrasound probe is away from the tip of the needle and toward the target blood vessel) and proceeds through step 136 as many times as is necessary until the tip of the needle is determined to have reached the target blood vessel, such as puncturing the blood vessel.

At step 136 of the method, the vascular access system 200 determines from the final ultrasound imaging that the tip of the needle is in the target blood vessel. This can be accomplished in any of a wide variety of ways. For example, the colocation of the tip of the needle and the target blood vessel can be determined using a targeting or identification algorithm, among other methods. Many ultrasound targeting or identification algorithms are known, and can be used for determining that the tip of the needle is in the target blood vessel.

At step 140 of the method, the vascular access system 200 reports the determination that the tip of the needle is in the target blood vessel, to a user via the user interface. The system may provide the report to a user - such as a patient or a clinician - via any mechanism, including but not limited to a visual display or any other method of notification. The information may be communicated by wired and/or wireless communication to another device. For example, the system may communicate the information to a mobile phone, computer, laptop, wearable device, and/or any other device configured to allow display and/or other communication of the information. According to one embodiment, the user interface is a component of the vascular access system 200. According to another embodiment, the user interface is a component of a device or system separate from the vascular access system 200.

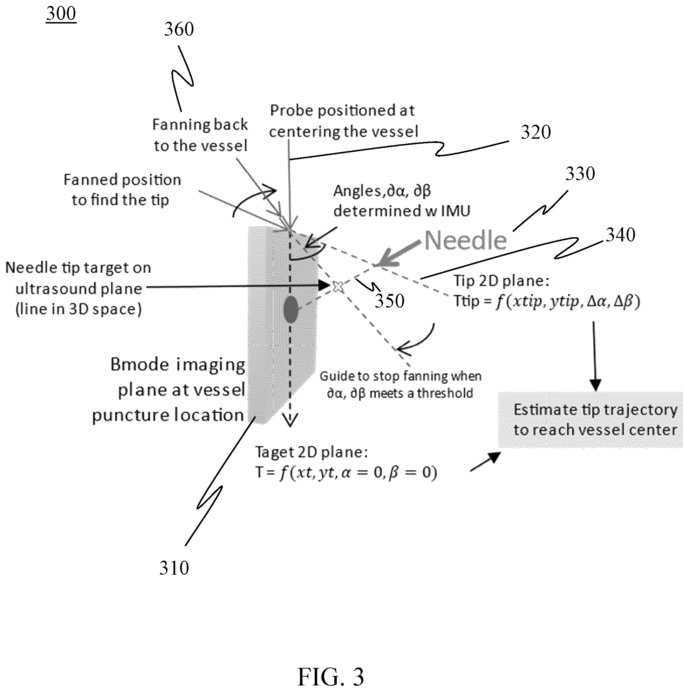

Referring to Fig. 3, in one embodiment, is a schematic representation 300 of out-of-plane vessel access using elevational probe tilting to track progression of the needle. According to an embodiment, the gray slab 310 represents cross-sectional ultrasound imaging plane of the ultrasound probe, and the central dark circle on the gray slab 310 indicates the cross section of the vessel at the desired puncture location. The elevational probe position 320 is depicted above the imaging plane, and the needle insertion is 330.

According to an embodiment, the probe or sensors associated therewith measure the elevational (α) and lateral (β) angle of the imaging plane. This measurement can, for example, be based on inclinometers that measure tip and tilt based on gravity, optical methods, mechanical methods, or by electronically steering a 2D imaging plane of a 3D ultrasound probe. When the ultrasound imaging plane is positioned at the desired vessel puncture cross section, the current elevational and lateral imaging plane position are set as the reference orientation, i.e. α=0, β=0. The lateral and depth coordinates of the vessel in the imaging plane are also stored (xt, yt).

Distal to the imaging plane, the needle is inserted into the skin surface and pushed a small distance roughly towards the intended vessel insertion location, while the imaging plane is tilted elevationally (α) towards the needle until the needle tip is detected. Compared to the traditional out-of-plane method using elevational translation instead of elevational tilt, a more orthogonal needle orientation can be achieved that enhances needle visibility. Also this example describes inserting the needle distal to the imaging plane as this is preferable for some applications, but for other vessels an approach from proximal direction may be preferred.

According to an embodiment, this elevationally tilted imaging plane orientation where the tip is detected is illustrated at 340 in Fig. 3. The moment the tip is detected, the change in elevational and lateral tilt (Δα, Δβ) compared to the reference orientation (α, β) is stored. The lateral and depth coordinates of the needle tip in the imaging plane are also stored (xtip, ytip).

According to an embodiment, the stored values α, β, xt, and yt - representing the target location - define a reference 3D location in the physical 3D space in the tissue. The stored values Δα, Δβ, xtip, and ytip - representing the needle tip location - define another 3D location in the same physical 3D space in the tissue. The straight line path between these two points can be calculated, and represents the ideal trajectory of the needle tip for further insertion towards the target (illustrated with dashed line 350).

According to an embodiment, the scanning plane is then gradually tilted back from the needle tip intersection plane (340) towards the vessel puncture location plane (350) in small steps (360). For any elevational tilt between Δα (first tip detection) and 0 (vessel puncture plane), the intersection point of the ideal trajectory within the current imaging plane can be calculated, and a target needle location marker is shown on the current live ultrasound image. As the clinician gradually inserts the needle deeper until it is visible in the new tilted imaging plane, the deviation from the ideal trajectory indicated by the target needle location marker is noticed, and small adjustments in needle orientation can be made while pushing the needle deeper.

According to an embodiment, one can keep track of the deviation from the optimal path and make an estimate on how far off-target the vessel puncture will be. When needle steepness is not correct the puncture could take place more proximal or distal than intended and with a different angle of approach. Lateral deviation could cause the needle to miss the vessel all together. Upon exceeding certain target deviation estimation thresholds warning messages could be issued to the UI, including suggestions to abort and retry.

According to an embodiment, for precise guidance it may be desirable to have small incremental steps in the elevational probe tilt, so multiple small adjustments can be made to the needle orientation during the insertion. An optional notification message can be provided when the amount of elevational probe tilting in a guidance step has exceeded a desired threshold, to help the clinician in taking sufficiently small guidance steps.

According to an embodiment, after several of above guidance steps the elevational tilt corresponding to the vessel puncture location will be reached (α=0). The lateral probe tilt β may at this point deviate slightly from its original β=0 orientation, but the target needle location marker will be at the location of the vessel cross section, and the final needle push to achieve puncture can be performed.

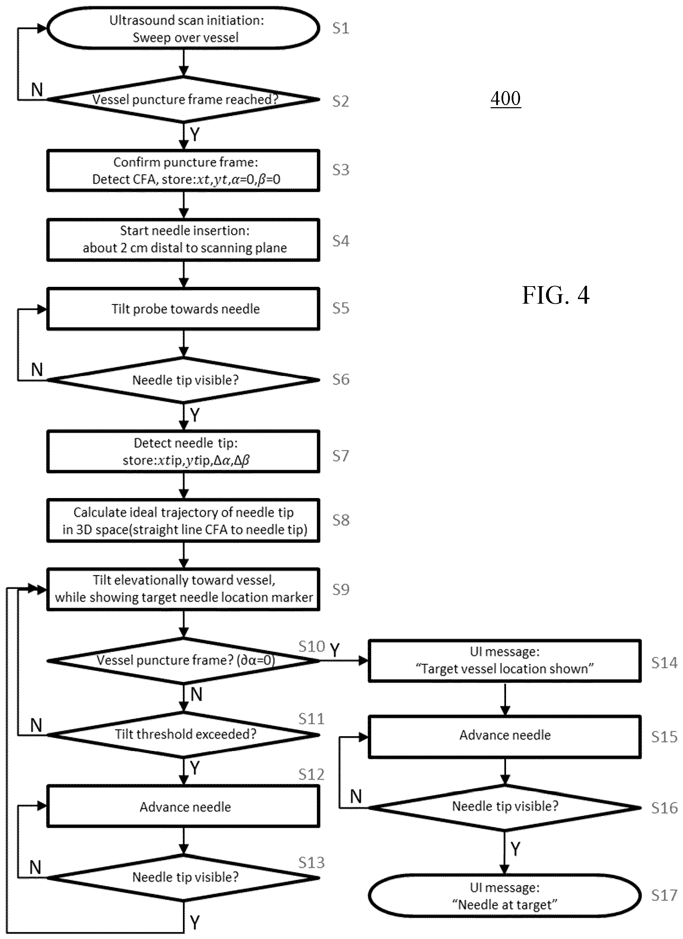

Referring to Fig. 4, in one embodiment, is a flowchart of a method 400 for guiding needle access to a target blood vessel using a vascular access system. Thus, method 400 shows out-of-plane vessel access using elevational probe tilting to track progression of needle. The methods described in connection with this figure is provided as an example only, and shall be understood not to limit the scope of the disclosure. The vascular access system can be any of the systems described or otherwise envisioned herein.

In step S1 of method 400, the clinician orients the probe to obtain a cross section of the vessel and sweeps over the vasculature in elevational directions (e.g., proximal, distal) to find the preferred location for the needle to enter the vessel. At step S2 of method 400, the system analyzes whether the vessel puncture frame has been identified or reached, comprising the target blood vessel.

One example of a vascular access application is Resuscitative Endovascular Balloon Occlusion of the Aorta (REBOA), which requires common femoral artery (CFA) access. Referring back to step S1, for a CFA puncture this would be approximately 2 cm proximal to the CFA bifurcation point.

The sweeping loop S1-S2 continues until the clinician has identified the optimal vessel puncture location. With the optimal vessel puncture location visualized the method progresses to step S3, where the clinician indicates the puncture location. This confirmation of the puncture frame can be done, for example, by pushing a button on the ultrasound system. Alternatively one could monitor the position tilt sensor in the probe to detect that the method has already progressed to step S5, and go back in the frame buffer to a frame before the probe was moved to retrospectively implement step S3. When the puncture frame is confirmed, the location of the target in the ultrasound image (xt, yt) is detected with a suitable algorithm, for example, and the probe orientation (α,β) is measured with a sensor. These parameters (xt, yt, α, β) are then stored for later use.

In step S4 the needle is inserted and advanced a small amount. For CFA access, the CFA is roughly 2 cm deep, and with a desired entry angle of 45 degrees the insertion point should be roughly 2 cm distal to the scanning plane.

In the S5-S6 loop, the probe is tilted elevationally towards the needle while keeping the aperture at the same location, until the needle tip becomes visible. This is a significant improvement over conventional workflows where the probe would be translated at constant tilt towards the needle. The advantage of tilting the probe rather than translating it, is that the angle between the scanning plane and the needle shaft gets closer to orthogonal and therefore the needle visibility gets much better. As soon as the needle tip is detected in step S6, the method proceeds to step S7 where both the tip location in the scanning plane and the current probe orientation (Δα, Δβ*,* xtip, ytip) are stored for later use.

In step S8 the system can now calculate the ideal trajectory of the needle tip in real 3D space, based on the data that was stored in steps S3 and S7.

Next, in step 9, the imaging plane is gradually tilted away from the needle tip and in the direction of the intended vessel puncture plane. Since the system knows the current orientation of the imaging plane (∂α*,* ∂β)*,* the system can calculate the point where the ideal trajectory of the needle tip intersects with the imaging plane and show a marker for the desired needle tip trajectory on that plane.

Steps S9/S10/S11 form a loop as the plane is tilted further away from the needle tip (i.e., ∂α is getting smaller) and in step S11 an optional notification message can be provided when the amount of elevational probe tilting in a guidance step has exceeded a desired threshold, to help the clinician in taking sufficiently small guidance steps.

At the desired tilt step size, the probe is held steady and the method enters loop S12/S13 where the needle is advanced until it is detected in the current imaging plane. The deviation of the needle tip from the ideal trajectory indicated by the target needle location marker can now be observed, and small adjustments in needle orientation can be made when inserting further.

The S9/S10/S11/S12/S13 loop repeats as in alternating steps the imaging plane is tilted closer to the vessel insertion frame (i.e., ∂α is getting smaller) and the needle is advanced. As the intended vessel puncture imaging plane is approached, ∂α will approach 0 and step S10 exits the loop to step S14 where a message can be shown on the UI indication that the targeted vessel puncture location is visualized in the imaging plane. Steps S15/S16/S17 present the last needle advancement into the vessel, with a UI message indicating when the target has been reached.

According to another embodiment, the vascular access system 200 provides out-of-plane needle insertion guidance with 3D ultrasound probe using a marker for desired needle location on an elevationally tilted imaging plane. According to an embodiment, the system employs a 3D ultrasound probe that is able to electronically steer the imaging plane in elevational direction. In this embodiment no additional sensor is needed to measure the elevational tilt α, and the lateral tilt β will be zero. Since electronic plane steering is rapid, the captured imaging frames can alternate between the vessel puncture imaging plane Δα=0, and the current needle tip tracking imaging frame ∂α, and these two images can be shown simultaneously side-by-side.

According to one embodiment, the elevational steering angle can be controlled by adjusting a knob on the ultrasound scanner as needed. One can also add push button activated fixed step sizes for the tilt to automate loop S9/S10/S11.

According to one embodiment, when coupled with algorithms to automatically detect the needle tip in the ultrasound imaging frame, loops S4/S5/S6, S9/S10/S11/S12/S13, and S15/S16 can be fully automated. The tilted imaging plane angle can be dynamically controlled in closed loop fashion to sweep back and forth and track the tip of the needle as it is inserted. In this workflow the clinician does not have to adjust the ultrasound machine, keeping both hands free to hold the probe and the needle.

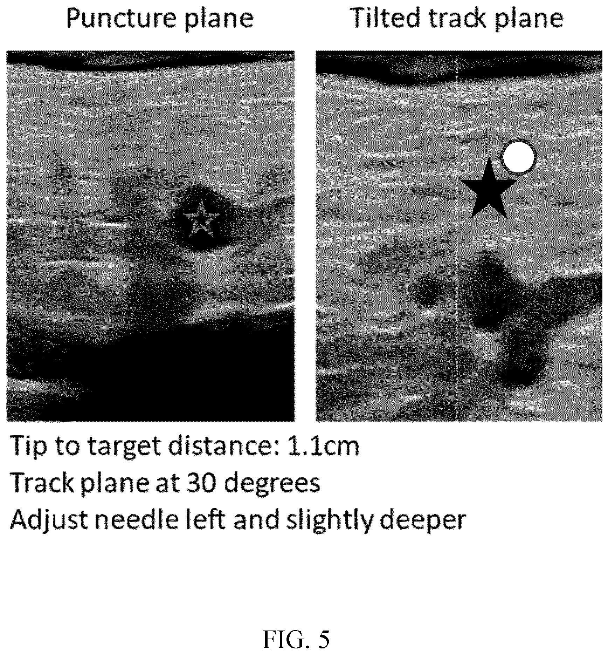

Referring to Fig. 5, in one embodiment, is a schematic representation of a user interface showing 3D probe needle guidance. The panel on the left has the orthogonal cross section plane through the desired vessel puncture location. The star in the left panel indicates the desired needle tip position (could be determined through AI algorithm or manual indication by physician). The right panel shows the electronically steered tilted imaging plane, with the star indicating the point where the ideal trajectory of the needle tip intersects with the titled imaging plane and the circle indicating a detected actual needle tip location.

According to an embodiment, the 3D probe could be a mechanically wobbled 2D probe. The wobble could either be a cyclical mechanical vibration such that angle is determined by timing of acquired frame, or an actuator that can statically position the transduced at the desired angle under electronic control.

Referring again to Fig. 2 is a schematic representation of a vascular access system 200. System 200 may be any of the systems described or otherwise envisioned herein, and may comprise any of the components described or otherwise envisioned herein. It will be understood that Fig. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, system 200 comprises or is in direct or indirect communication with an imaging modality 270. The imaging modality can be any modality sufficient to obtain imagery utilized by the ultrasound system 200 to obtain ultrasound imagery and identify target anatomy. The imaging modality can be, for example, an ultrasound system such as EPIQ^{®} system, Philips Lumify^{®}, and Philips Affinity^{®}, among many others.

According to an embodiment, storage 260 of system 200 may store one or more algorithms, modules, and/or instructions to carry out one or more functions or steps of the methods described or otherwise envisioned herein. For example, storage 260 may comprise, among other instructions or data, ultrasound imaging data 262, angle data 263, trajectory generation instructions 264, and/or reporting instructions 265.

According to an embodiment, ultrasound imaging data 262 is any or all of the ultrasound imaging data obtained by the clinician and/or system using the imaging modality 270. For example, the ultrasound imaging data 262 can comprise the initial ultrasound imaging of the patient or subject. The initial ultrasound imaging includes ultrasound imaging of the target blood vessel, including while a tip of a needle is positioned at a first position relative to the target blood vessel. According to an embodiment, the ultrasound imaging data 262 also comprises the updated ultrasound imaging, obtained after the needle is moved from an initial first position to a second position and while or after the elevational angle of the ultrasound probe is adjusted toward the needle. According to an embodiment, the ultrasound imaging data 262 also comprises the final ultrasound imaging, which determines that the tip of the needle is in the target blood vessel

According to an embodiment, angle data 263 is any of the angle data received or obtained by the system during the course of the process or methods described or otherwise envisioned herein. For example, the angle data 263 can comprise the elevational (α) angle and lateral (β) angle of the imaging plane, i.e., of the ultrasound imaging probe 272 at the time of obtaining the initial ultrasound imaging. According to an embodiment, the angle data 263 can comprise the updated elevational angle (α) and an updated lateral angle (β) of the ultrasound probe at the new position, after tilting the elevational angle of the probe.

According to an embodiment, the angle data 263 can comprise the lateral coordinates of the target (xt) and the depth coordinates of the target (yt). The angle data 263 can also comprise the updated lateral coordinate (xt) and updated depth coordinate (yt) for the detected tip of the needle, obtained from the updated ultrasound imaging, after adjusting the elevational angle of the ultrasound probe while keeping the aperture of the ultrasound probe at the same location. The angle data 263 can also comprise other data, as described or otherwise envisioned herein.

According to an embodiment, trajectory generation instructions 264, direct the system to generate a trajectory between a second position of the needle tip and the identified location of the target blood vessel. The trajectory can be generated in a wide variety of different ways. According to an embodiment, the system generates the trajectory using the updated elevational angle (α), updated lateral angle (β), updated lateral coordinate (xt) of the detected tip of the needle, the updated depth coordinate (yt) of the detected tip of the needle, and the identified location of the target blood vessel. According to an embodiment, the generated trajectory comprises a needle location marker.

According to an embodiment, reporting instructions 265 direct the system to provide information to the user, such as a clinician, via a user interface. The provided information can be any of the information as described or otherwise envisioned herein, including but not limited to the determination that the needle tip has reached the target, such as puncturing the target blood vessel. This may comprise an alert, and/or may comprise ultrasound imaging information including a display of the needle tip in the target blood vessel. The system may provide the information to a user via any mechanism, including but not limited to a visual display, an audible notification, a page, or any other method of notification. The information may be communicated by wired and/or wireless communication to another device. For example, the system may communicate the information to a mobile phone, computer, laptop, wearable device, and/or any other device configured to allow display and/or other communication of the information.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for needle access to a target blood vessel, comprising:
receiving (112), from an ultrasound imaging probe (272) of a vascular access system (200), ultrasound imaging including ultrasound imaging of the target blood vessel, while a tip of the needle is positioned at a first position relative to the target blood vessel;
receiving (114), from one or more sensors (280) of the vascular access system (200), information about an initial elevational angle and an initial lateral angle of the ultrasound imaging probe at the time of obtaining the received ultrasound imaging;
receiving (116) an identification of a location of the target blood vessel in the received ultrasound imaging;
adjusting (118), after the tip of the needle is moved to a second position toward the location of the target blood vessel, the elevational angle of the ultrasound probe toward the needle while obtaining updated ultrasound imaging, until the tip of the needle is detected in the updated ultrasound imaging;
receiving (120) an updated elevational angle and an updated lateral angle of the ultrasound imaging probe at the time of obtaining the updated ultrasound imaging;
receiving (122) a lateral coordinate and depth coordinate for the detected tip of the needle;
generating (124), using the updated elevational angle, the updated lateral angle, the lateral coordinate of the detected tip of the needle, the depth coordinate of the detected tip of the needle, and the identified location of the target blood vessel, a trajectory between the second position and the identified location of the target blood vessel;
adjusting (126), after the trajectory between the second position and the identified location of the target blood vessel is generated, the elevational angle of the ultrasound probe away from the tip of the needle and toward the target blood vessel;
displaying (128) the generated trajectory on a user interface, the generated trajectory comprising a needle location marker;
receiving (134), after the tip of the needle is moved to a third position based on the displayed generated trajectory, final ultrasound imaging; and
determining (136), from the final ultrasound imaging, that the tip of the needle is in the target blood vessel.

2. The method of claim 1, wherein receiving an identification of a location of the target blood vessel in the received ultrasound imaging comprises either receiving an identification from a clinician via a user interface or an identification of the target blood vessel by an ultrasound algorithm.

3. The method of claim 1 or 2, wherein receiving ultrasound imaging including ultrasound imaging of the target blood vessel comprises receiving a sweep of a region comprising the target blood vessel.

4. The method of any of claims 1 to 3, wherein an aperture of the ultrasound probe is kept at a same location while the elevational angle of the ultrasound probe is adjusted toward the needle.

5. The method of any of claims 1 to 4, further comprising the step of reporting (138) to the clinician via the user interface, the determination that the tip of the needle is in the target blood vessel.

6. The method of any of claims 1 to 5, further comprising:
determining (130), after the elevational angle of the ultrasound probe is adjusted away from the tip of the needle and toward the target blood vessel, that the adjustment exceeds a predetermined threshold of the elevational angle; and
providing (132), via the user interface, an indication that the adjustment exceeds a predetermined threshold of the elevational angle.

7. The method of any of claims 1 to 6, wherein the steps of adjusting (126), displaying (128), receiving (134) are repeated until the step of determining that the tip of the needle is in the target blood vessel.

8. A vascular access system (200) configured to enable needle access to a target blood vessel, comprising:
a needle comprising a needle tip;
an ultrasound probe (272) configured to obtain ultrasound imaging including ultrasound imaging of the target blood vessel, while the tip of the needle is positioned at a first position relative to the target blood vessel;
one or more sensors (280) configured to obtain information about an initial elevational angle and an initial lateral angle of the ultrasound imaging probe at the time of obtaining ultrasound imaging;
a user interface (240); and
a processor (220) configured to: (i) receive, via the ultrasound probe, ultrasound imaging including ultrasound imaging of the target blood vessel; (ii) receive, from the one or more sensors, information about an initial elevational angle and an initial lateral angle of the ultrasound imaging probe at the time of obtaining ultrasound imaging; (iii) receive an identification of a location of the target blood vessel in the received ultrasound imaging; (iv) adjust, after the tip of the needle is moved to a second position toward the location of the target blood vessel, the elevational angle of the ultrasound probe toward the needle while obtaining updated ultrasound imaging, until the tip of the needle is detected in the updated ultrasound imaging; (v) receive an updated elevational angle and an updated lateral angle of the ultrasound imaging probe at the time of obtaining the updated ultrasound imaging; (vi) receive a lateral coordinate and depth coordinate for the detected tip of the needle; (vii) generate, using the updated elevational angle, the updated lateral angle, the lateral coordinate of the detected tip of the needle, the depth coordinate of the detected tip of the needle, and the identified location of the target blood vessel, a trajectory between the second position and the identified location of the target blood vessel; (viii) adjust, after the trajectory between the second position and the identified location of the target blood vessel is generated, the elevational angle of the ultrasound probe away from the tip of the needle and toward the target blood vessel; (ix) display the generated trajectory on the user interface; (x) receive, after the tip of the needle is moved to a third position based on the displayed generated trajectory, final ultrasound imaging; and (xi) determine, from the final ultrasound imaging, that the tip of the needle is in the target blood vessel.

9. The vascular access system of claim 8, wherein receiving an identification of a location of the target blood vessel in the received ultrasound imaging comprises either receiving an identification from a clinician via a user interface or an identification of the target blood vessel by an ultrasound algorithm.

10. The vascular access system of claim 8 or 9, wherein receiving ultrasound imaging including ultrasound imaging of the target blood vessel comprises receiving a sweep of a region comprising the target blood vessel.

11. The vascular access system of any of claims 8 to 10, wherein an aperture of the ultrasound probe is kept at a same location while the elevational angle of the ultrasound probe is adjusted toward the needle.

12. The vascular access system of any of claims 8 to 11, wherein the user interface is further configured to report to the clinician the determination that the tip of the needle is in the target blood vessel.

13. The vascular access system of any of claims 8 to 12, wherein the processor is further configured to: determine, after the elevational angle of the ultrasound probe is adjusted away from the tip of the needle and toward the target blood vessel, that the adjustment exceeds a predetermined threshold of the elevational angle; and provide, via the user interface, an indication that the adjustment exceeds a predetermined threshold of the elevational angle.

14. The vascular access system of any of claims 8 to 13, wherein the processor is further configured to repeat the steps of adjusting, displaying, and receiving until the step of determining that the tip of the needle is in the target blood vessel.

15. A non-transitory machine-readable medium (260) storing instructions which, when executed by a processor (220) of a vascular access system of any of claims 8 to 14, cause the processor to perform the method of any of claims 1 to 7.
